# EUROPEAN PATENT APPLICATION

(11) **EP 4 772 106 A1**
(43) Date of publication of application: **08.07.2026**
(21) Application number: 24940940.0
(22) Date of filing: 27.09.2024
(51) Int. Cl.: A61B 5/00, A61B 5/024, A61B 5/389

(54) **PHYSIOLOGICAL SIGNAL MONITORING DEVICE**

(71) Applicant: Ascend Technology Limited, Hong Kong 999077 (HK)
(72) Inventor: SU, Qi, Shenzhen, Guangdong 518108 (CN); ZHOU, Xin, Shenzhen, Guangdong 518108 (CN); ZHANG, Xiaoxiao, Shenzhen, Guangdong 518108 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2024/121894
(87) International publication number: WO 2026/065125

(57) **Abstract**

The present disclosure provides a device for monitoring physiological signals. The device for monitoring physiological signals includes a wearable body. The wearable body includes: one or more electrodes configured to contact human skin to collect human physiological signals, each of the one or more electrodes is formed by printing conductive silicone ink on a substrate; and a base body configured to carry the one or more electrodes and position the one or more electrodes at a target portion of a human body.

## Description

### TECHNICAL FIELD

The present disclosure relates to the field of signal monitoring, and in particular to a device for monitoring physiological signals.

### BACKGROUND

A heart rate belt, as a device for monitoring physiological signals, may include two electrocardiographic electrodes. An electrocardiographic signal is determined based on a potential difference between positions of the two electrocardiographic electrodes. However, in heart rate belt products, the electrocardiographic electrodes for collecting electrocardiographic signals are typically metal electrodes (e.g., silver patch electrodes). Due to poor corrosion resistance and durability of the metal electrodes, the service life of the product is shortened.

Therefore, it is necessary to provide a device for monitoring physiological signals to improve the durability of the electrodes on the heart rate belt, thereby extending the service life of the heart rate belt.

### SUMMARY

One or more embodiments of the present disclosure provide a device for monitoring physiological signals, the device for monitoring physiological signals includes: a wearable body including: one or more electrodes configured to contact human skin to collect human physiological signals, each of the one or more electrodes is formed by printing conductive silicone ink on a substrate; and a base body configured to carry the one or more electrodes and position the one or more electrodes at a target portion of a human body.

In some embodiments, the substrate of each of the one or more electrodes is stacked on a surface of the base body close to the human skin.

In some embodiments, the substrate of each of the one or more electrodes and the base body are connected by adhesion.

In some embodiments, the substrate of each of the one or more electrodes and the base body are connected in a side-by-side manner.

In some embodiments, the base body and the substrate are formed by integrated weaving of one or more yarns.

In some embodiments, the wearable body further includes a waterproof layer. At least a portion of the one or more electrodes is connected to the base body through the waterproof layer.

In some embodiments, the waterproof layer includes a first waterproof film, the first waterproof film is stacked on a surface of the base body close to the human skin, and two side surfaces of the first waterproof film are adhesively connected to the base body and the one or more electrodes, respectively.

In some embodiments, the waterproof layer further includes a second waterproof film, the second waterproof film is stacked on a side of the first waterproof film close to the human skin, and the second waterproof film is adhesively connected to a peripheral side of at least a portion of the one or more electrodes.

In some embodiments, the waterproof layer includes a waterproof insulating yarn, the base body includes an elastic yarn, and the waterproof layer and the base body are formed by integrated weaving.

In some embodiments, the waterproof layer is stacked on a surface of the base body close to the human skin, and the substrate of each of the one or more electrodes is located on a side of the waterproof layer away from the base body.

In some embodiments, the waterproof layer and the base body are woven in a side-by-side manner, the base body is connected to a peripheral side of the waterproof layer, and the substrate of each of the one or more electrodes is located on a surface of the waterproof layer close to the human skin.

In some embodiments, the substrate of each of the one or more electrodes includes an insulating yarn, and the substrate and the waterproof layer are formed by integrated weaving.

In some embodiments, the substrate, the waterproof layer, and the base body are woven in a side-by-side manner, and the waterproof layer isolates the substrate from the base body.

In some embodiments, each of the one or more electrodes further includes an underlayer, and at least a portion of the underlayer is located between the waterproof layer and a portion of the substrate.

In some embodiments, the one or more electrodes include a first electrode and a second electrode, the first electrode and the second electrode are configured to measure electrocardiographic signals, and the first electrode and the second electrode are located on two sides of a median sagittal plane.

In some embodiments, the one or more electrodes include a first electrode and a second electrode, the first electrode and the second electrode are configured to collect electromyographic signals of a same muscle, and the first electrode and the second electrode are arranged at intervals along a direction of muscle fibers of the muscle.

In some embodiments, two metal snaps are fixed disposed on the base body, one of the two metal snaps is electrically connected to the first electrode, the other of the two metal snaps is electrically connected to the second electrode, the two metal snaps implement data transmission between the first electrode and the second electrode and a processing circuit, and the processing circuit is detachably connected to the two metal snaps in a magnetic attraction manner.

In some embodiments, the wearable body further includes one or more conductive layers, the one or more conductive layers are located between the two metal snaps and the one or more electrodes, and the two metal snaps are electrically connected to the one or more electrodes through the one or more conductive layers.

In some embodiments, the base body is brushed.

In some embodiments, a conductivity of the one or more electrodes is in a range of 0.1 S/cm to 0.3 S/cm.

### BRIEF DESCRIPTION OF THE DRAWINGS

The present disclosure is further illustrated in terms of exemplary embodiments. These exemplary embodiments are described in detail with reference to the drawings. These embodiments are not limiting, and in these embodiments, the same numbering indicates the same structure, wherein:
FIG. 1 is a schematic diagram illustrating an application scenario of a device for monitoring physiological signals according to some embodiments of the present disclosure;
FIG. 2A is a schematic diagram illustrating a structure of an inner surface of a device for monitoring physiological signals according to some embodiments of the present disclosure;
FIG. 2B is a schematic diagram illustrating a structure of an outer surface of a device for monitoring physiological signals according to some embodiments of the present disclosure;
FIG. 3 is a schematic diagram illustrating a front view of a device for monitoring physiological signals according to some embodiments of the present disclosure;
FIG. 4 is a schematic diagram illustrating a front view of a device for monitoring physiological signals according to some other embodiments of the present disclosure;
FIG. 5 is a schematic diagram illustrating a front view of a device for monitoring physiological signals according to some other embodiments of the present disclosure;
FIG. 6 is a schematic diagram illustrating a structure of an inner surface of a device for monitoring physiological signals according to some other embodiments of the present disclosure;
FIG. 7 is a schematic diagram illustrating a front view of a device for monitoring physiological signals according to some other embodiments of the present disclosure;
FIG. 8 is a schematic diagram illustrating another front view of a device for monitoring physiological signals according to some other embodiments of the present disclosure;
FIG. 9 is a schematic diagram illustrating another front view of a device for monitoring physiological signals according to some other embodiments of the present disclosure;
FIG. 10 is a schematic diagram illustrating another structure of an inner surface of a device for monitoring physiological signals according to some other embodiments of the present disclosure;
FIG. 11 is a schematic diagram illustrating another front view of a device for monitoring physiological signals according to some other embodiments of the present disclosure;
FIG. 12 is a schematic diagram illustrating another front view of a device for monitoring physiological signals according to some other embodiments of the present disclosure;
FIG. 13 is a schematic diagram illustrating another front view of a device for monitoring physiological signals according to some other embodiments of the present disclosure;
FIG. 14 is a schematic diagram illustrating another front view of a device for monitoring physiological signals according to some other embodiments of the present disclosure;
FIG. 15 is a schematic diagram illustrating positions where one or more electrodes are located on a human body according to some embodiments of the present disclosure; and
FIG. 16 is a schematic diagram illustrating another front view of a device for monitoring physiological signals according to some other embodiments of the present disclosure.

### DETAILED DESCRIPTION

To more clearly illustrate the technical solutions of the embodiments of the present disclosure, the drawings used in the description of the embodiments are briefly introduced below. Obviously, the drawings in the following description are merely some examples or embodiments of the present disclosure. For those skilled in the art, the present disclosure may be applied to other similar scenarios based on these drawings without creative effort. Unless obviously obtained from the context or the context illustrates otherwise, the same numeral in the drawings refers to the same structure or operation.

It should be understood that the terms "system", "device", "unit", and/or "module" used herein are a method for distinguishing components, elements, parts, sections, or assemblies of different levels. However, if other words are able to achieve the same purpose, the words may be replaced by other expressions.

As used in the present disclosure and the appended claims, the singular forms "a," "an," and "the" include plural referents unless the content clearly dictates otherwise. Generally, the terms "include" and "comprise" only indicate the inclusion of explicitly identified steps and elements. These steps and elements do not constitute an exclusive list. A method or device may also include other steps or elements.

In the description of the present disclosure, it should be understood that the terms "first", "second", "third", "fourth", etc., are used for descriptive purposes only and are unable to be understood as indicating or implying relative importance or implicitly indicating the quantity of the indicated technical features. Thus, features defined with "first", "second", "third", "fourth" are able to explicitly or implicitly include at least one of the features. In the description of the present disclosure, the term "a plurality of" means at least two, for example, two, three, etc., unless otherwise explicitly and specifically defined.

In the present disclosure, unless otherwise explicitly specified and defined, terms such as "connect" and "fix" should be understood broadly. For example, the term "connect" is able to refer to a fixed connection, a detachable connection, or an integrated connection. It may be a mechanical connection or an electrical connection. It may be a direct connection or an indirect connection through an intermediate medium. It may be the internal communication between two elements or the interaction relationship between two elements, unless otherwise explicitly defined. Those skilled in the art may understand the specific meanings of the above terms in the present disclosure according to specific situations.

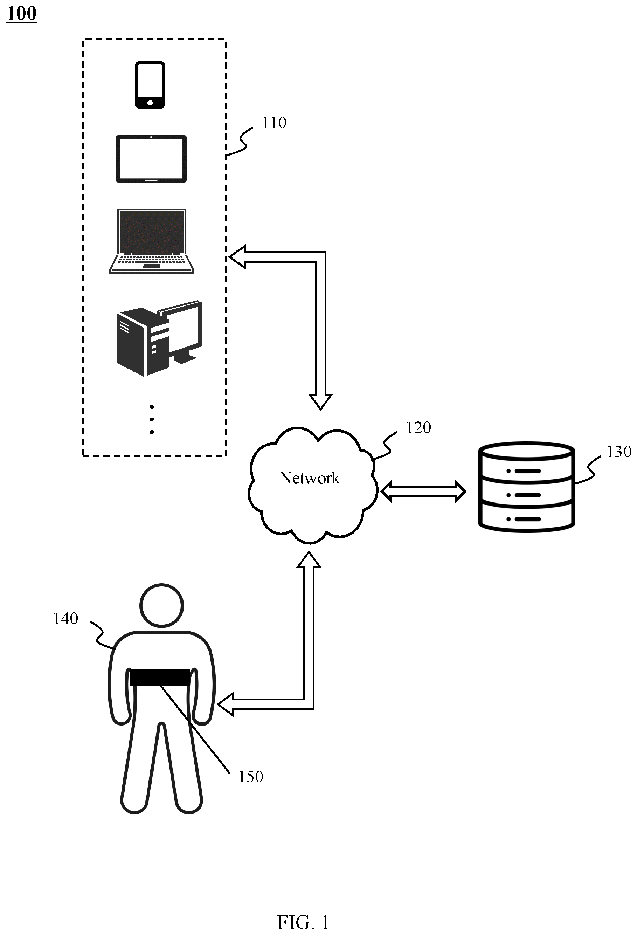

FIG. 1 is a schematic diagram illustrating an application scenario of a device for monitoring physiological signals according to some embodiments of the present disclosure. As shown in FIG. 1, in some embodiments, an application scenario 100 of a device for monitoring physiological signals (hereinafter referred to as the application scenario 100) may include a terminal device 110, a network 120, a storage device 130, a monitored object 140, and a device 150 for monitoring physiological signals. In some embodiments, various components in the application scenario 100 (e.g., the terminal device 110, the storage device 130, the device 150 for monitoring physiological signals) may be connected and/or communicate with each other via the network 120 (e.g., a wireless connection, a wired connection, or a combination thereof).

The terminal device 110 refers to a device and/or software used by a user associated with the application scenario 100. The user associated with the application scenario 100 includes, but is not limited to, the monitored object 140, a physician (e.g., a clinician, a radiation therapist), a nurse, etc. For example, the terminal device 110 may be a device or software for controlling the device 150 for monitoring physiological signals, the user issues a control instruction to the device 150 for monitoring physiological signals through the terminal device 110, thereby controlling the device 150 for monitoring physiological signals to collect physiological signals of the monitored object 140. For example, the terminal device 110 may send the control instruction input by the user to the device 150 for monitoring physiological signals via the network 120 to control the device 150 for monitoring physiological signals to collect physiological signals of the monitored object 140. In some embodiments, the terminal device 110 may obtain the physiological signals of the monitored object 140 collected by the device 150 for monitoring physiological signals via the network 120. In some embodiments, the terminal device 110 may be a mobile device, a tablet computer, a laptop computer, a desktop computer, or other devices with input and/or output functions, or any combination thereof.

A physiological signal refers to a bioelectrical signal (e.g., an electrocardiographic signal, an electromyographic signal) of an object (e.g., the monitored object 140) collected based on a signal acquisition device (e.g., the device 150 for monitoring physiological signals). The physiological signal may be a mixed signal including a noise signal (e.g., a motion artifact signal, a static electricity signal) and a clean physiological signal. The clean physiological signal refers to a real physiological signal of the object (e.g., the monitored object 140) obtained after filtering out the noise signal.

The network 120 may connect various components (e.g., the terminal device 110, the storage device 130, the device 150 for monitoring physiological signals) of the application scenario 100 and/or connect the application scenario 100 with an external resource portion. The network 120 may enable communication between various components of the application scenario 100 and between the application scenario 100 and other external portions, thereby facilitating an exchange of data and/or information. For example, the terminal device 110 may obtain the physiological signals of the monitored object 140 collected by the device 150 for monitoring physiological signals via the network 120. As another example, the storage device 130 may obtain physiological signal data of the monitored object 140 collected by the device 150 for monitoring physiological signals via the network 120 and store the physiological signal data.

In some embodiments, the network 120 may be any form of a wired or wireless network, or any combination thereof. Merely by way of example, the network 120 may include a cable network, a wired network, a fiber optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near field communication (NFC) network, etc., or any combination thereof. In some embodiments, the network 120 may include at least one network access point, and at least one component of the application scenario 100 may connect to the network 120 via the network access point to exchange data and/or information. For example, collected physiological signals and other data may be transmitted via the network 120.

The storage device 130 may store data, instructions, and/or any other information. In some embodiments, the storage device 130 may store data obtained from the device 150 for monitoring physiological signals and/or the terminal device 110. For example, the storage device 130 may store the physiological signals collected by the device 150 for monitoring physiological signals. In some embodiments, the storage device 130 may include a mass storage, a removable storage, a volatile read-write memory, a read-only memory (ROM), etc., or any combination thereof. An exemplary mass storage may include a magnetic disk, an optical disk, a solid-state disk, etc. In some embodiments, the storage device 130 may be implemented on a cloud platform. Merely by way of example, the cloud platform may include a private cloud, a public cloud, a hybrid cloud, a community cloud, a distributed cloud, an internal cloud, a multi-layer cloud, etc., or any combination thereof.

In some embodiments, the storage device 130 may be connected to the network 120 to communicate with at least one other component in the application scenario 100. The at least one component in the application scenario 100 may access data, instructions, or other information stored in the storage device 130 via the network 120. In some embodiments, the storage device 130 may be directly connected to or communicate with one or more components (e.g., the device 150 for monitoring physiological signals, the terminal device 110) in the application scenario 100. In some embodiments, the storage device 130 may be a portion of the device 150 for monitoring physiological signals and/or the terminal device 110.

The monitored object 140 refers to a monitored object of the device 150 for monitoring physiological signals, for example, a user of the device 150 for monitoring physiological signals or a patient, an athlete, an experimenter, etc., whose physiological signals need to be collected.

The device 150 for monitoring physiological signals refers to a device for collecting or monitoring the physiological signals of the monitored object 140. The device 150 for monitoring physiological signals may be positioned at at least one portion (e.g., the chest, the back, the waist) of a body of the monitored object 140 to collect the physiological signals of the monitored object 140. In some embodiments, the device 150 for monitoring physiological signals may be configured to monitor the physiological signals of the monitored object 140 in a motion scenario. For example, in the motion scenario, the device 150 for monitoring physiological signals may be positioned at the chest of the monitored object 140 to collect electrocardiographic signals of the monitored object 140 during exercise, thereby guiding the monitored object 140 to exercise scientifically based on the electrocardiographic signals and changes in the electrocardiographic signals. As another example, in the motion scenario, the device 150 for monitoring physiological signals may be positioned at a certain muscle position (e.g., the pectoralis major, the biceps brachii, etc.) of the monitored object 140 to collect electromyographic signals of the muscle of the monitored object 140, thereby guiding the monitored object 140 to exercise scientifically (e.g., preventing muscle damage during exercise) based on the electromyographic signals and changes in the electromyographic signal. In some embodiments, the device for monitoring physiological signals may also be configured to monitor the physiological signals of the monitored object 140 in other scenarios (e.g., a sleep state, fetal heart monitoring for pregnant women, etc.). For example, in the sleep scenario, the device 150 for monitoring physiological signals may be positioned at the chest of the monitored object 140 to collect the electrocardiographic signals of the monitored object 140 during sleep, thereby determining a sleep quality of the monitored object 140 based on the electrocardiographic signals and changes in the electrocardiographic signals. As another example, when the monitored object 140 is a pregnant woman, the device 150 for monitoring physiological signals may be configured to monitor a fetal heart rate (i.e., a fetal heart signal) of the pregnant woman, and determine a health status of a fetus based on the fetal heart signal and changes of the fetal heart signal.

In some embodiments, the device 150 for monitoring physiological signals may be a strip structure (e.g., a heart rate strap for measuring a heart rate). The device 150 for monitoring physiological signals may include a base body (e.g., a strap that is able to be wrapped and positioned on the body) and one or more electrodes. The base body may be configured to position the device 150 for monitoring physiological signals at the at least one portion of the body of the monitored object 140. The one or more electrodes may be arranged on an inner surface of the base body close to the human skin, and configured to fit to the at least one portion of the body of the monitored object 140 to collect the physiological signals (e.g., electric potentials at positions where the one or more electrodes are located) of the monitored object 140. In some embodiments, the device 150 for monitoring physiological signals may be directly worn on the at least one portion of the body of the monitored object 140, or may be combined with a wearable device to fit to the at least one portion of the body of the monitored object 140. For example, the device 150 for monitoring physiological signals may be arranged on an inner surface of clothing.

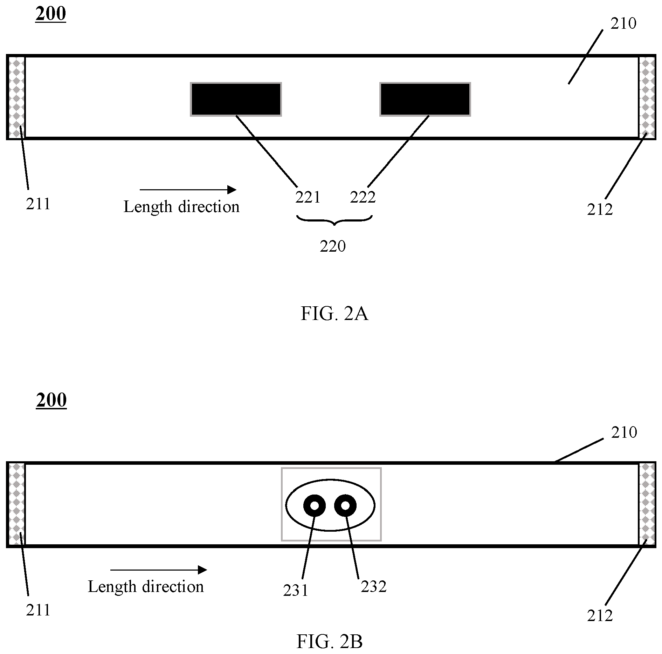

In some embodiments, the device 150 for monitoring physiological signals may possess an independent power source. The device 150 for monitoring physiological signals may transmit collected data (e.g., the physiological signals) to other components (e.g., the storage device 130, the terminal device 110) in a wired or wireless (e.g., Bluetooth, Wi-Fi, etc.) manner. In some embodiments, one or more components of the application scenario 100 may be a portion of the device 150 for monitoring physiological signals. For example, the device 150 for monitoring physiological signals may include the storage device 130, etc. More descriptions regarding the device 150 for monitoring physiological signals may be found in FIG. 2A to FIG. 14 and related descriptions thereof.

In some embodiments, the application scenario 100 may further include a processing circuit (not shown in FIG. 1, the processing circuit may be configured in the device 150 for monitoring physiological signals or the terminal device 110). The processing circuit may determine physiological data based on electrical signals collected by a plurality of electrodes. The physiological data refers to data determined based on the physiological signals and reflecting bioelectrical characteristics of a monitored object (e.g., the monitored object 140). For example, when the physiological signals are the electrocardiographic signals, the physiological data may be electrocardiogram data. In some embodiments, the processing circuit may provide a feedback to the monitored object 140 based on changes in the physiological signals or the physiological data. For example, the processing circuit may remind a user (e.g., the monitored object 140) to adjust an exercise mode or intensity based on the electrocardiographic signals or changes in the electrocardiographic signals during exercise, so as to exercise scientifically. In some embodiments, the processing circuit may generate reminder information based on changes in the physiological signals or the physiological data and send the reminder information to the terminal device 110. For example, according to the changes in the physiological signals or the physiological data, the terminal device 110 may perform voice prompts, generate vibrations, etc., to remind the user.

It should be noted that the above descriptions regarding the application scenario 100 are merely for illustration and explanation, and do not limit the applicable scope of the present disclosure. For those skilled in the art, various modifications and changes may be made to the application scenario 100 under the guidance of the present disclosure. However, these modifications and changes are still within the scope of the present disclosure.

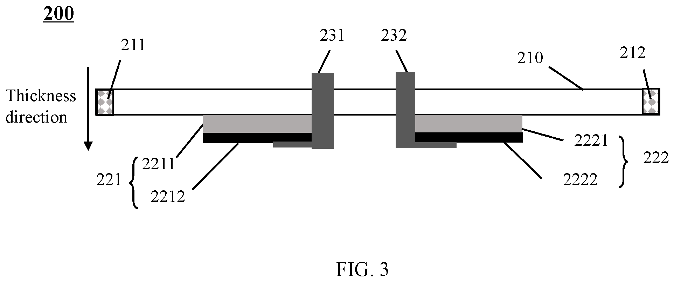

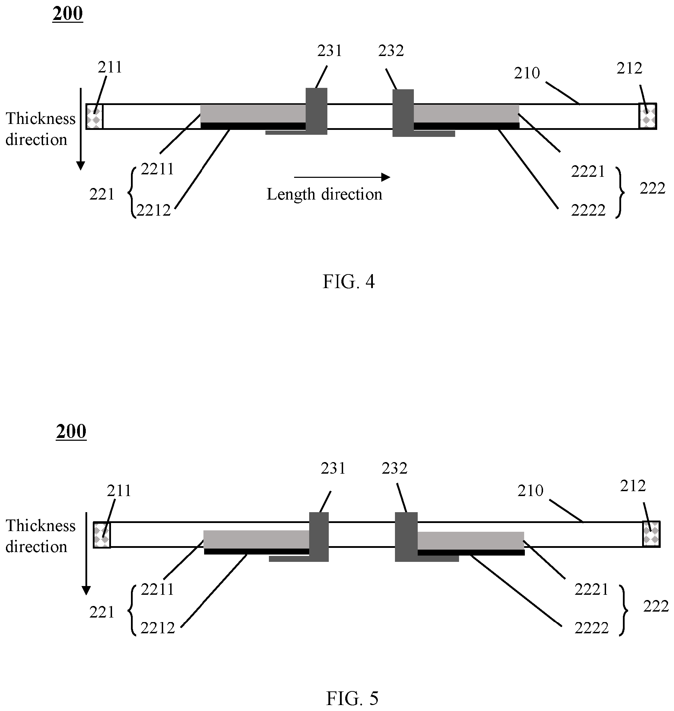

FIG. 2A is a schematic diagram illustrating a structure of an inner surface of a device for monitoring physiological signals according to some embodiments of the present disclosure; FIG. 2B is a schematic diagram illustrating a structure of an outer surface of a device for monitoring physiological signals according to some embodiments of the present disclosure; FIG. 3 is a schematic diagram illustrating a front view of a device for monitoring physiological signals according to some embodiments of the present disclosure; FIG. 4 is a schematic diagram illustrating a front view of a device for monitoring physiological signals according to some other embodiments of the present disclosure; and FIG. 5 is a schematic diagram illustrating a front view of a device for monitoring physiological signals according to some other embodiments of the present disclosure. FIG. 3, FIG. 4, and FIG. 5 are the front views of the device for monitoring physiological signals when placed flat on a horizontal plane (e.g., when the outer surface or inner surface is placed parallel to a tabletop/ground), and subsequent other front views are similar.

In some embodiments, the device for monitoring physiological signals may include a wearable body. The wearable body refers to a portion of the device for monitoring physiological signals that is worn on a portion (e.g., the chest, the waist, etc.) of the body of a user (e.g., the monitored object 140). In some embodiments, the wearable body may be directly worn on a portion of the body of the user. For example, the wearable body is worn on the body of the user through a base body (e.g., a base body 210 described later). In some embodiments, the wearable body may be combined with a wearable device to be worn on a portion of the body of the user. For example, the wearable body may be arranged on an inner surface of clothing, thereby being worn on the body of the user.

In some embodiments, as shown in FIG. 2A, the wearable body 200 includes a base body 210 and one or more electrodes 220.

The base body 210 is configured to carry the one or more electrodes 220 and position the one or more electrodes 220 at a target portion of a human body. The target portion of a human body refers to a portion of the body where physiological signals are to be measured. For example, when the device for monitoring physiological signals is configured to measure electrocardiographic signals, the base body 210 may position the one or more electrodes 220 at the chest of the human body. At this time, the target portion of a human body corresponds to the chest. As another example, when the device for monitoring physiological signals is configured to measure the electromyographic signals, the base body 210 may position the one or more electrodes 220 at a portion of a muscle to be measured. For example, when measuring the electromyographic signals of a pectoralis major, the one or more electrodes are positioned at the pectoralis major. At this time, the target portion of the human body is the portion of the muscle to be measured. As another example, when the device for monitoring physiological signals is configured to measure the fetal heart signal, the base body 210 may position the one or more electrodes 220 at the abdomen of the pregnant woman. At this time, the target portion of the human body is the abdomen.

In some embodiments, as shown in FIG. 2A, the base body 210 may be a strip shape.

In some embodiments, as shown in FIG. 2A, two ends of the base body 210 along a length direction may be respectively configured with a connecting member 211 and a connecting member 212. When the connecting member 211 and the connecting member 212 are connected (e.g., buckled), the base body 210 may surround and fit a wearing portion (e.g., the chest, waist, etc.) of the user (e.g., the monitored object 140). In some embodiments, the connecting member may be a connecting buckle. For example, the connecting member may be a button, a snap fastener, a magnetic buckle, etc.

In some embodiments, a material of the base body 210 may include a plant fiber (e.g., a cotton fiber, a hemp fiber, etc.), an animal fiber (e.g., wool, etc.), a synthetic fiber (e.g., an acrylic fiber, a polyester fiber, a spandex fiber, etc.), a modified chemical fiber, etc. For example, the base body 210 may be woven from a yarn made of the plant fiber (e.g., the cotton yarn).

In some embodiments, the base body 210 may include an elastic yarn (e.g., a cotton yarn, a spandex yarn, etc.), i.e., the yarn used for the base body 210 is the elastic yarn, and the base body 210 is formed by weaving the elastic yarn.

In some embodiments, the base body 210 may be brushed to make the wearable body 200 more comfortable when fitting the skin.

The one or more electrodes 220 are configured to contact human skin to collect human physiological signals (e.g., the electrocardiographic signals, the electromyographic signals). In some embodiments, as shown in FIG. 2A, the one or more electrodes 220 include a first electrode 221 and a second electrode 222. The first electrode 221 and the second electrode 222 are arranged at intervals along a length direction of the wearable body 200.

In some embodiments, the first electrode 220 may include a substrate and a coating. For example, as shown in FIG. 3, the first electrode 221 includes a substrate 2211 and a coating 2212, and the second electrode 222 includes a substrate 2221 and a coating 2222.

The substrate is configured to carry the coating. The coating is printed on a surface of the substrate facing away from the base body. At this time, the base body, the substrate, and the coating are sequentially stacked along a thickness direction of the wearable body 200. A material of the coating is a conductive material and is configured to collect the physiological signals. In some embodiments, a material of the substrate is a low-elastic textile. For example, the material of the substrate may be a low-elastic fabric, a woven fabric, or the like. By setting the material of the substrate as the low-elastic textile, the substrate may possess better stability, thereby facilitating the printing of the coating. In some embodiments, the material of the substrate may be the same as or similar to the material of the base body 210. For example, the material of the substrate may include a plant fiber (e.g., a cotton fiber, a hemp fiber, etc.), an animal fiber (e.g., wool, etc.), a synthetic fiber (e.g., an acrylic fiber, a polyester fiber, a spandex fiber, etc.), a modified chemical fiber, etc.

In some embodiments, the coating may be conductive silicone ink. Each of the one or more electrodes is formed by printing the coating (e.g., conductive silicone ink) on the substrate. That is, the first electrode 221 is formed by printing the conductive silicone ink on the substrate 2211, and the second electrode 222 is formed by printing the conductive silicone ink on the substrate 2221. In some embodiments, each of the one or more electrodes 220 may not include the substrate. In this case, the one or more electrodes 220 are formed by directly printing the coating at a preset position on the surface of the base body 210 close to the human skin (i.e., a preset region on the surface of the base body 210 for carrying the one or more electrodes). In some embodiments, a preparation process of the conductive silicone ink may be as follows. First, conductive particles are added to an organic solvent, and the organic solvent is used to fully disperse the conductive particles. Then, silicone is added, and the silicone forms a network to wrap and confine the fully dispersed conductive particles, thereby forming the conductive silicone ink. At this time, the conductive silicone ink is in a liquid or fluid state, and is printed on a surface of the substrate. After the organic solvent evaporates, solid electrodes are formed. The conductive particles herein may include metal particles, carbon particles, or any combination thereof. In some embodiments, the conductive silicone ink possesses good chemical stability and is able to resist a variety of solvents and chemicals. The conductive silicone ink also possesses good biocompatibility, thereby enabling the device for monitoring physiological signals to be suitable for medical devices and biosensors. In addition, the conductive silicone ink also possesses a function of increasing moisture, i.e., the conductive silicone ink may reduce sweat evaporation.

In some embodiments, compared with traditional metal electrodes (e.g., silver electrodes) that are completely exposed to air and susceptible to a corrosion, the conductive particles in the present disclosure are confined in the silicone, which is able to reduce contact with the air. Therefore, the conductive silicone ink possesses strong corrosion resistance and a longer service life than the traditional metal electrodes. In addition, when the conductive particles are carbon particles, the conductive silicone ink possesses strong corrosion resistance and a longer service life because the carbon particles are more stable and chemically inert. In some scenarios, the one or more electrodes may also be formed by molding solid conductive silicone. However, the one or more electrodes obtained in this manner have a relatively large thickness and high hardness. In contrast, liquefying the conductive silicone ink and then printing the conductive silicone ink on the substrate may enable a thickness of the conductive silicone ink relatively thin, thereby enabling the wearable body 200 better flexibility and elasticity. Furthermore, since the uniformity of dispersion of the conductive particles affects the conductivity of the conductive silicone ink, traditional solid silicone possesses a very high viscosity and poor uniformity of dispersion of the conductive particles, resulting in poor conductivity. In contrast, the preparation process of the conductive silicone ink may make the dispersion of the conductive particles more uniform, resulting in better conductivity.

In some embodiments, to enable the wearable body 200 to accurately and effectively collect the physiological signals of the user, a conductivity of each of the one or more electrodes formed by printing the conductive silicone ink on the substrate is in a range of 0.1 S/cm to 0.3 S/cm. Merely by way of example, to improve the accuracy of the wearable body 200 in collecting the physiological signals of the user, the conductivity of each of the one or more electrodes formed by printing the conductive silicone ink on the substrate is 0.125 S/cm. In some embodiments, to improve the comfort of wearing the wearable body 200, the thickness of the conductive silicone ink printed on the substrate may be in a range of 200 micrometers to 1000 micrometers.

In some embodiments, by printing the conductive silicone ink on the substrate to form the one or more electrodes, the conductive silicone ink and the substrate are in a state that is difficult to separate. At the same time, after the conductive silicone ink is coated, the conductive silicone ink penetrates into the substrate and fuses with the substrate, so that the conductive silicone ink electrode possesses a higher strength.

In some embodiments, the substrate of each of the one or more electrodes is stacked on the surface of the base body 210 close to the human skin (i.e., the inner surface of the base body), and the coating of each of the one or more electrodes is located on a surface of the substrate away from the base body 210. At this time, the base body 210, the substrate of each of the one or more electrodes, and the coating of each of the one or more electrodes are sequentially stacked along the thickness direction of the wearable body 200. For example, as shown in FIG. 3, the substrate 2211 of the first electrode 221 is stacked on the inner surface of the base body 210 (i.e., a surface close to the human skin), the coating 2212 of the first electrode 221 is located on the surface of the substrate 2211 away from the base body 210, and the base body 210, the substrate 2211, and the coating 2212 are sequentially stacked along the thickness direction. Similarly, the substrate 2221 of the second electrode 222 is stacked on the inner surface of the base body 210, the coating 2222 of the second electrode 222 is located on the surface of the substrate 2221 away from the base body 210, and the base body 210, the substrate 2221, and the coating 2222 are sequentially stacked along the thickness direction.

In some embodiments, the substrate of each of the one or more electrodes (the substrate 2211 of the first electrode 221, the substrate 2221 of the second electrode 222) and the base body 210 may be connected by adhesion. For example, a side of the substrate of each of the one or more electrodes facing the base body 210 may be provided with a backing adhesive, and the substrate is adhered to the base body 210 through the backing adhesive.

In some embodiments of the present disclosure, the substrate is stacked on the inner surface of the base body, and the conductive silicone ink is printed on the surface of the substrate away from the base body to form the electrode, which can enable the one or more electrodes to fit the human skin to accurately measure physiological signals of the human body. In some embodiments, the substrate of the electrode and the base body are connected by adhesion, so that the electrode and the base body can be processed and produced separately and then adhered after respective production is completed, thereby improving production efficiency.

In some embodiments, the substrate of each of the one or more electrodes and the base body 210 may be connected in a side-by-side manner. At this time, the substrate of the electrode is embedded in the base body 210. In some embodiments, the substrate of the electrode may be completely embedded in the base body 210 (i.e., the electrode penetrates through the base body 210 along the thickness direction). At this time, the surface of the substrate away from the coating is flush with an outer surface of the base body 210 (the surface of the base body 210 away from the human skin), and the base body 210 surrounds a peripheral side of the electrode. For example, as shown in FIG. 4, the substrate 2211 of the first electrode 221 and the substrate 2221 of the second electrode 222 are distributed at intervals along the length direction, and the substrate 2211 and the substrate 2221 are completely embedded in the base body 210. A surface of the substrate 2211 away from the coating 2212 and a surface of the substrate 2221 away from the coating 2222 are flush with the outer surface of the base body 210, and the base body 210 surrounds peripheral sides of the first electrode 221 and the second electrode 222. The base body 210, the first electrode 221, and the second electrode 222 are spliced side by side along the length direction. In some embodiments, the substrate of the electrode may be partially embedded in the base body 210. For example, as shown in FIG. 5, the substrate 2211 of the first electrode 221 and the substrate 2221 of the second electrode 222 are partially embedded in the base body 210. The base body 210 surrounds a peripheral side of the substrate 2211 and the surface of the substrate 2211 away from the coating 2212, and the base body 210 surrounds a peripheral side of the substrate 2221 and the surface of the substrate 2221 away from the coating 2222.

In some embodiments of the present disclosure, by setting the substrate and the base body to be connected in the side-by-side manner, an overall thickness of the wearable body 200 can be reduced, and flexibility can be improved.

In some embodiments, the base body and the substrate are formed by mixed weaving or integrated weaving of one or more yarns.

The integrated weaving refers to a process of continuously weaving one or more yarns (e.g., the cotton yarn, the polyester yarn, etc.) to obtain an integral fabric. Exemplarily, an integrated weaving process of the base body and the substrate may be as follows: based on the yarns included in the base body and the substrate respectively (e.g., the base body includes the cotton yarn, and the substrate includes the polyester yarn), the base body and the substrate are continuously woven according to a preset positional relationship between the base body and the substrate (e.g., the positional relationship between the base body and the substrate as shown in FIG. 3, FIG. 4, or FIG. 5) to obtain a formed base body and substrate. During the weaving process, when a transition is performed between the base body and the substrate, a transition weaving technique (e.g., tuck weaving) needs to be used. Furthermore, after obtaining a woven base body and substrate based on the above integrated weaving process, the coating may be printed on the surface of the substrate that faces the human skin (e.g., printing the conductive silicone ink on the surface of the substrate), thereby obtaining a complete wearable body.

In some embodiments of the present disclosure, the integrated weaving can improve the stability of the connection between the substrate and the base body.

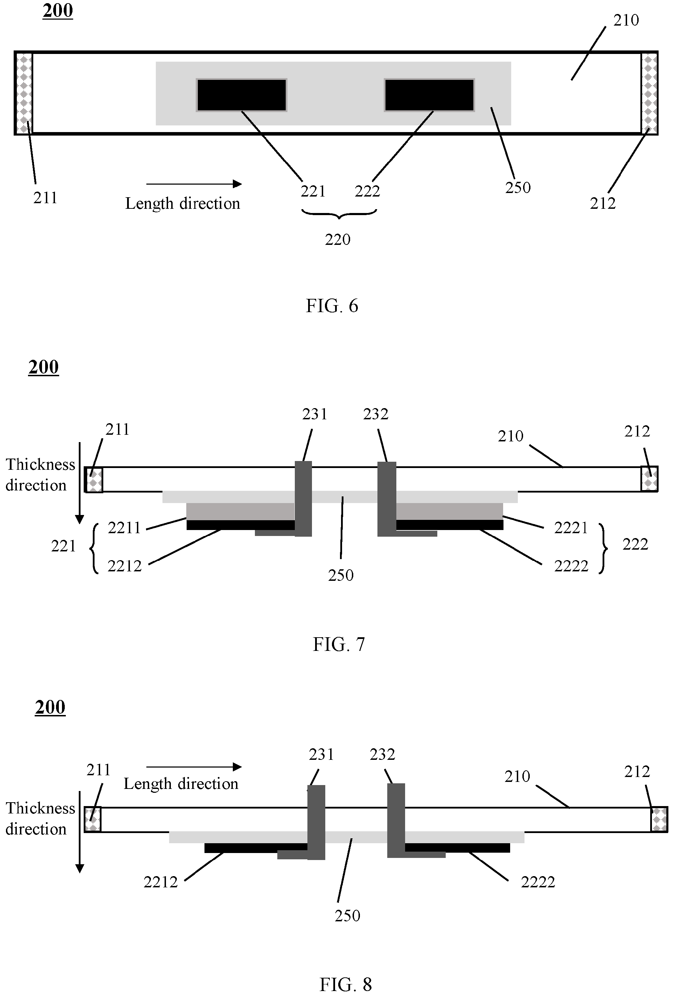

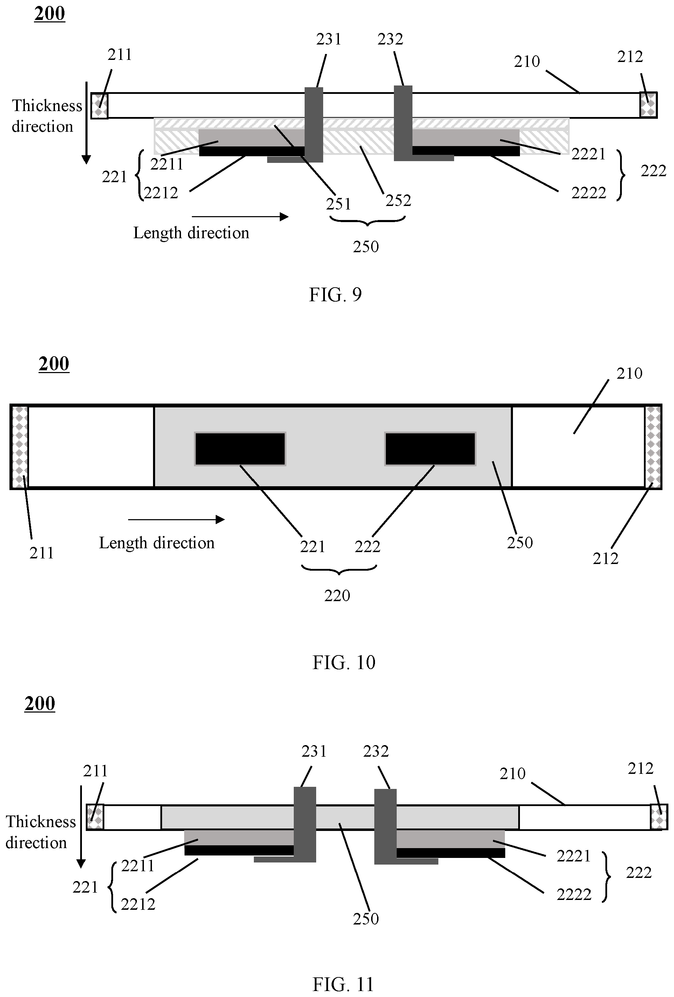

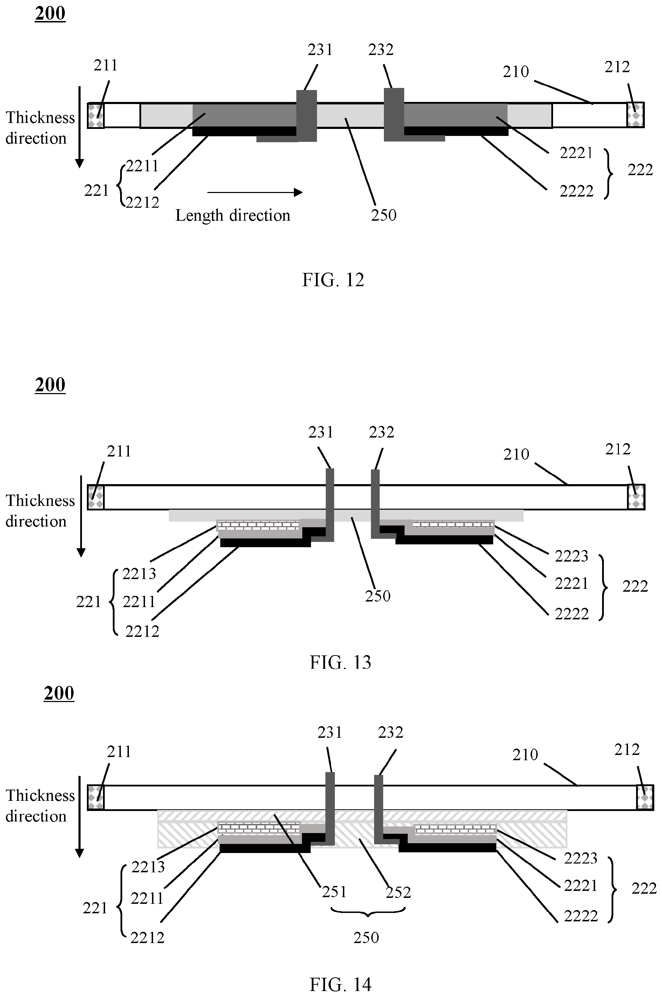

FIG. 6 is a schematic diagram illustrating a structure of an inner surface of a device for monitoring physiological signals according to some other embodiments of the present disclosure. FIG. 7 is a schematic diagram illustrating a front view of a device for monitoring physiological signals according to some other embodiments of the present disclosure. FIG. 8 is a schematic diagram illustrating another front view of a device for monitoring physiological signals according to some other embodiments of the present disclosure. FIG. 9 is a schematic diagram illustrating another front view of a device for monitoring physiological signals according to some other embodiments of the present disclosure. FIG. 10 is a schematic diagram illustrating another structure of an inner surface of a device for monitoring physiological signals according to some other embodiments of the present disclosure. FIG. 11 is a schematic diagram illustrating another front view of a device for monitoring physiological signals according to some other embodiments of the present disclosure. FIG. 12 is a schematic diagram illustrating another front view of a device for monitoring physiological signals according to some other embodiments of the present disclosure. FIG. 13 is a schematic diagram illustrating another front view of a device for monitoring physiological signals according to some other embodiments of the present disclosure. FIG. 14 is a schematic diagram illustrating another front view of a device for monitoring physiological signals according to some other embodiments of the present disclosure.

In some embodiments, a wearable body includes a waterproof layer, and at least a portion of one or more electrodes is connected to a base body through the waterproof layer. For example, as shown in FIG. 6 and FIG. 7, the wearable body 200 further includes a waterproof layer 250, and the first electrode 221 and the second electrode 222 are connected to the base body 210 through the waterproof layer 250.

When the base body 210 is soaked with liquid (e.g., when a user exercises, sweat soaks into the base body 210), the base body 210 becomes conductive. At this time, in order to prevent an abnormal conduction between the first electrode 221 and the second electrode 222 through the base body 210, the first electrode 221 and the second electrode 222 need to be insulated from the base body 210. Based on this, a material of the waterproof layer 250 may be an insulating waterproof material. For example, the material of the waterproof layer 250 may be an insulating rubber, a silicone, etc.

In some embodiments, when the wearable body includes the waterproof layer, each of the one or more electrodes may only include the coating (i.e., each of the one or more electrodes does not include the substrate), and the coating is directly printed on a surface of the waterproof layer away from the base body. For example, the conductive silicone ink is directly printed on a portion of the surface of the waterproof layer away from the base body. As shown in FIG. 8, the coating 2212 is directly printed on a portion of the surface of the waterproof layer 250 away from the base body 210, the coating 2212 serves as the first electrode 221, the coating 2222 is directly printed on a portion of the surface of the waterproof layer 250 away from the base body 210 (the coating 2212 and the coating 2222 are printed on the surface of the waterproof layer 250 at intervals along the length direction), and the coating 2222 serves as the second electrode 222.

In some embodiments, the waterproof layer includes a first waterproof film, the first waterproof film is stacked on a surface of the base body close to the human skin, and two side surfaces of the first waterproof film are adhesively connected to the base body and the one or more electrodes, respectively. As shown in FIG. 9, the waterproof layer 250 includes a first waterproof film 251, the first waterproof film 251 is stacked on the surface of the base body 210 close to the human skin, and two side surfaces of the first waterproof film 251 are adhesively connected to the base body 210 and the one or more electrodes (the first electrode 221 and the second electrode 222), respectively. For example, the two surfaces of the first waterproof film 251 may be provided with the backing adhesives, respectively, and the first waterproof film 251 is adhesively connected to the base body 210 and the one or more electrodes (the first electrode 221 and the second electrode 222) through the backing adhesives.

The first waterproof film 251 is located between the base body 210 and the one or more electrodes (the first electrode 221 and the second electrode 222). The first waterproof film 251 may block liquid between the base body 210 and the one or more electrodes, thereby achieving waterproofing of the one or more electrodes and avoiding the abnormal conduction between the first electrode 221 and the second electrode 222.

In some embodiments, the waterproof layer further includes a second waterproof film, the second waterproof film is stacked on a side of the first waterproof film close to the human skin, and the second waterproof film is adhesively connected to a peripheral side of at least a portion of the one or more electrodes. For example, as shown in FIG. 9, the waterproof layer 250 further includes a second waterproof film 252, the second waterproof film 252 is stacked on a side of the first waterproof film 251 close to the human skin, and the second waterproof film 252 is adhesively connected to the peripheral side of the one or more electrodes (including the first electrode 221 and the second electrode 222). A surface of the first waterproof film 251 faces the human skin, and at least a portion of the first waterproof film 251 may contact the human skin. When sweat is present on a surface of the skin, the sweat may flow onto the first waterproof film 251, the sweat on the first waterproof film 251 (especially a portion between the first electrode 221 and the second electrode 222 along the length direction) may also cause an abnormal conduction between the first electrode 221 and the second electrode 222. At this time, by providing the second waterproof film 252, the second waterproof film 252 surrounds the peripheral sides of the first electrode 221 and the second electrode 222, thereby further achieving waterproofing for the two electrodes.

In some embodiments, a material of the waterproof film (including the first waterproof film and the second waterproof film) is a waterproof insulating film. For example, the material of the waterproof film is a rubber film, etc. In some embodiments, the material of the waterproof film may be a polyurethane (PU) film.

In some embodiments of the present disclosure, by providing the first waterproof film 251 and the second waterproof film 252, electrode waterproofing can be achieved, the abnormal conduction between the two electrodes due to immersion can be prevented. By selecting the polyurethane film as the material of the waterproof layer, due to the high Young's modulus of the polyurethane film, a more stable combination with a data interface can be formed, and noise during the signal acquisition can be reduced.

In some embodiments, the waterproof layer 250 includes a waterproof insulating yarn (e.g., an acrylic yarn, a spandex yarn, etc.), the base body 210 includes an elastic yarn (e.g., a polyester yarn, a nylon yarn, etc.), and the waterproof layer 250 and the base body 210 are formed by integrated weaving.

Exemplarily, an integrated weaving process for the waterproof layer and the base body may be: based on the yarns respectively included in the base body and the waterproof layer (the base body includes the elastic yarn, and the waterproof layer includes the waterproof insulating yarn), weaving the base body and the waterproof layer according to a positional relationship between the base body and the waterproof layer (e.g., a positional relationship between the base body and the waterproof layer shown in FIG. 6 and FIG. 7, or a positional relationship between the base body and the waterproof layer shown in FIG. 10 or FIG. 11) to obtain a formed base body and waterproof layer. When a transition is performed between the base body and the waterproof layer during the weaving process, a transition weaving technique (e.g., tuck weaving) needs to be used. Furthermore, after obtaining a woven base body and waterproof layer, the one or more electrodes may be positioned at a preset position on an inner surface of the waterproof layer to complete a combination of the base body, the one or more electrodes, and the waterproof layer included in the wearable body. Exemplarily, the positioning manner described above may be: using a yarn (e.g., an insulating yarn or the waterproof insulating yarn, etc.) to sew the one or more electrodes onto the surface of the waterproof layer (e.g., using the yarn to sew a fabric substrate of each of the one or more electrodes onto the inner surface of the waterproof layer, thereby achieving a conduction between the one or more electrodes and the waterproof layer), or, using an adhesive to adhere the one or more electrodes onto the surface of the waterproof layer.

In some embodiments, the waterproof layer is stacked on the surface of the base body close to the human skin, and the substrate of each of the one or more electrodes is located on a side of the waterproof layer away from the base body. For example, as shown in FIG. 6 and FIG. 7, the waterproof layer 250 is stacked on the surface of the base body 210 close to the human skin (i.e., the inner surface of the base body), and the substrate of each of the one or more electrodes (including the substrate 2211 of the first electrode 221 and the substrate 2221 of the second electrode 222) is located on a side of the waterproof layer 250 away from the base body 210.

Exemplarily, an integrated weaving process for the base body 210 and the waterproof layer 250 as shown in FIG. 6 and FIG. 7 may include: using the elastic yarn to weave and form the base body 210; using the waterproof insulating yarn to weave and form the waterproof layer 250 on a preset waterproof region (a region where the preset waterproof layer is located) of the inner surface of the base body 210. Furthermore, in a preset electrode region (a region on the surface of the waterproof layer preset for carrying the one or more electrodes) on the inner surface of the waterproof layer 250, the substrate 2211 of the first electrode 221 and the substrate 2221 of the second electrode 222 are woven onto the inner surface of the waterproof layer 250 using a low-elasticity yarn. The integrated weaving process further includes: printing the coating 2212 of the first electrode 221 on a surface of the substrate 2211 away from the waterproof layer 250, and printing the coating 2222 of the second electrode 222 on a surface of the substrate 2221 away from the waterproof layer 250, thereby obtaining a complete wearable body.

In some embodiments, the waterproof layer and the base body are woven in the side-by-side manner, the base body is connected to a peripheral side of the waterproof layer, and the substrate of each of the one or more electrodes is located on the surface of the waterproof layer close to the human skin. For example, as shown in FIG. 10 and FIG. 11, the waterproof layer 250 and the base body 210 are woven in a side-by-side manner (at this time, the waterproof layer 250 penetrates through the base body 210 along the thickness direction), the base body 210 is connected to a peripheral side of the waterproof layer 250, and the substrate of each of the one or more electrodes (including the substrate 2211 of the first electrode 221 and the substrate 2221 of the second electrode 222) is located on a surface of the waterproof layer 250 close to the human skin (i.e., the inner surface).

Exemplarily, an integrated weaving process for the base body 210 and the waterproof layer 250 as shown in FIG. 10 and FIG. 11 may be as follows: based on the yarns respectively included in the base body 210 and the waterproof layer 250 (e.g., the base body 210 includes the elastic yarn, and the waterproof layer 250 includes the waterproof insulating yarn), the base body 210 and the waterproof layer 250 are continuously woven according to a positional relationship between the base body 210 and the waterproof layer 250 shown in FIG. 10 and FIG. 11, so as to obtain the base body 210 and the waterproof layer 250 as shown in FIG. 10 and FIG. 11. When a transition is performed between the base body 210 and the waterproof layer 250 during the weaving process, a transition weaving technique (e.g., the tuck knitting) needs to be used. Furthermore, after the woven base body 210 and waterproof layer 250 are obtained, the one or more electrodes (including the first electrode 221 and the second electrode 222) may be position at the preset electrode region of the inner surface of the waterproof layer 250 to complete a combination of the base body 210, the one or more electrodes, and the waterproof layer 250 included in the wearable body 200.

In some embodiments, the substrate of each of the one or more electrodes includes an insulating yarn (e.g., the cotton yarn, the spandex yarn, etc.), and the substrate and the waterproof layer are formed by integrated weaving.

Exemplarily, taking the structure of the wearable body shown in FIG. 6 and FIG. 7, or shown in FIG. 10 and FIG. 11 as an example, an integrated weaving process for the substrate of each of the one or more electrodes (including the substrate 2211 of the first electrode 221 and the substrate 2221 of the second electrode 222) and the waterproof layer 250 may be as follows: the waterproof insulating yarn is used to weave and form the waterproof layer 250; in the preset electrode region on the inner surface of the waterproof layer 250, the low-elasticity yarn is used to weave the substrate of each of the one or more electrodes (including the substrate 2211 of the first electrode 221 and the substrate 2221 of the second electrode 222) onto the inner surface of the waterproof layer 250. Furthermore, the conductive silicone ink may be printed on surfaces of the woven substrate 2211 and substrate 2221 to form the first electrode 221 and the second electrode 222.

In some embodiments, the substrate, the waterproof layer, and the base body are woven in the side-by-side manner along the length direction, and the waterproof layer isolates the substrate from the base body. For example, as shown in FIG. 12, a substrate (including the substrate 2211 of the first electrode 221 and a substrate 2221 of the second electrode 222), the waterproof layer 250, and the base body 210 are woven in the side-by-side manner along the length direction, and in the length direction, the waterproof layer 250 isolates the substrate from the base body 210.

Exemplarily, an integrated weaving process for the substrate (including the substrate 2211 of the first electrode 221 and the substrate 2221 of the second electrode 222), the waterproof layer 250, and the base body 210 as shown in FIG. 12 may be as follows: based on the yarns respectively included in the substrate, the waterproof layer 250, and the base body 210 (e.g., the substrate includes the low-elasticity yarn, the waterproof layer 250 includes the waterproof insulating yarn, and the base body 210 includes the elastic yarn), the substrate, the waterproof layer 250, and the base body 210 are continuously woven according to a positional relationship between the substrate, the waterproof layer 250, and the base body 210 shown in FIG. 12, so as to obtain the substrate, the base body 210, and the waterproof layer 250 as shown in FIG. 12. When a transition is performed between the base body 210 and the waterproof layer 250, and between the waterproof layer 250 and the substrate during the weaving process, a transition weaving technique (e.g., the tuck weaving) needs to be used. Furthermore, after a woven substrate, base body 210, and waterproof layer 250 are obtained, the conductive silicone ink may be printed on the substrate to obtain the complete wearable body 200.

In some embodiments, each of the one or more electrodes further includes an underlayer, and at least a portion of the underlayer is located between the waterproof layer and a portion of the substrate. For example, as shown in FIG. 13, the first electrode 221 includes an underlayer 2213, and the second electrode 222 includes an underlayer 2223; at least a portion of the underlayer 2213 is located between the waterproof layer 250 and the substrate 2211, and at least a portion of the underlayer 2223 is located between the waterproof layer 250 and the substrate 2221.

In some embodiments, as shown in FIG. 14, when the waterproof layer 250 includes the first waterproof film 251 and the second waterproof film 252, the underlayer 2213 is located between the first waterproof film 251 and a portion of the substrate 2211, and the underlayer 2223 is located between the first waterproof film 251 and a portion of the substrate 2221. It is able to be understood that a portion of a surface of the substrate connected to the first waterproof film 251 is provided with a backing adhesive, and the substrate is adhesively connected to the first waterproof film 251 through the backing adhesive.

In some embodiments, a material of the underlayer may be different from the material of the substrate. For example, the material of the substrate may be a woven fabric with low elasticity, and the material of the underlayer may be a cotton yarn with high elasticity.

In some embodiments, the material of the underlayer may be the same as the material of the substrate. For example, the materials of both the substrate and the underlayer may be the woven fabric.

In some embodiments, when each of the one or more electrodes includes the underlayer, the one or more electrodes, the waterproof layer, and the base body may be formed by integrated weaving. Exemplarily, taking the structure of the wearable body shown in FIG. 13 as an example, an integrated weaving process for the one or more electrodes, the base body, and the waterproof layer may be as follows: the elastic yarn is used to weave and form the base body 210; the waterproof insulating yarn is used to weave and form the waterproof layer 250 on the preset waterproof region of the inner surface of the base body 210; in the preset electrode region of the waterproof layer 250, based on the yarns respectively included in the substrate (including the substrate 2211 of the first electrode 221 and the substrate 2221 of the second electrode 222) and the underlayer (including the underlayer 2213 of the first electrode 221 and the underlayer 2223 of the second electrode 222), the substrate and the underlayer are continuously weaved, thereby obtaining the combination of the substrate, the waterproof layer, and the base body as shown in FIG. 13. When a transition is performed between the substrate and the underlayer during the weaving process, a transition weaving technique (e.g., the tuck weaving) needs to be used. Furthermore, the coating is printed on the surface of the substrate (the conductive silicone ink is printed on surfaces of the substrate 2211 and the substrate 2221), thereby completing the combination of the base body 210, the one or more electrodes 220, and the waterproof layer 250 included in the wearable body 200.

In some embodiments of the present disclosure, a surface of each of the one or more electrodes facing away from the waterproof layer may be recessed inward relative to an inner surface of the wearable body, the one or more electrodes are caused to fail to continuously contact the human skin, thereby affecting the measurement of the physiological signals. Based on this, by providing the underlayer, the underlayer can cause a surface of a portion of the one or more electrodes to be flush with or even protrude from the inner surface of the base body, the one or more electrodes are ensured to continuously contact the human body when the wearable body is worn on the body surface of the user, thereby ensuring the quality of physiological signals measured by the one or more electrodes.

Different from forming the one or more electrodes by printing the conductive silicone ink on the substrate as described above, in some embodiments, the one or more electrodes may also adopt other structural forms. In some embodiments, a metal material may be used to prepare the one or more electrodes, e.g., each of the one or more electrodes may be a silver patch. The silver patch electrode is adhesively connected to the base body (or the waterproof layer), and the silver patch electrode contacts the human skin to collect human physiological signals. To prevent a corrosion and an oxidation of the silver patch electrode, the conductive silicone ink may be coated on a surface of the silver patch electrode close to the human skin, the conductive silicone ink may serve both a conductive function and a protective function for the silver patch electrode (e.g., preventing the corrosion and the oxidation of the silver patch electrode).

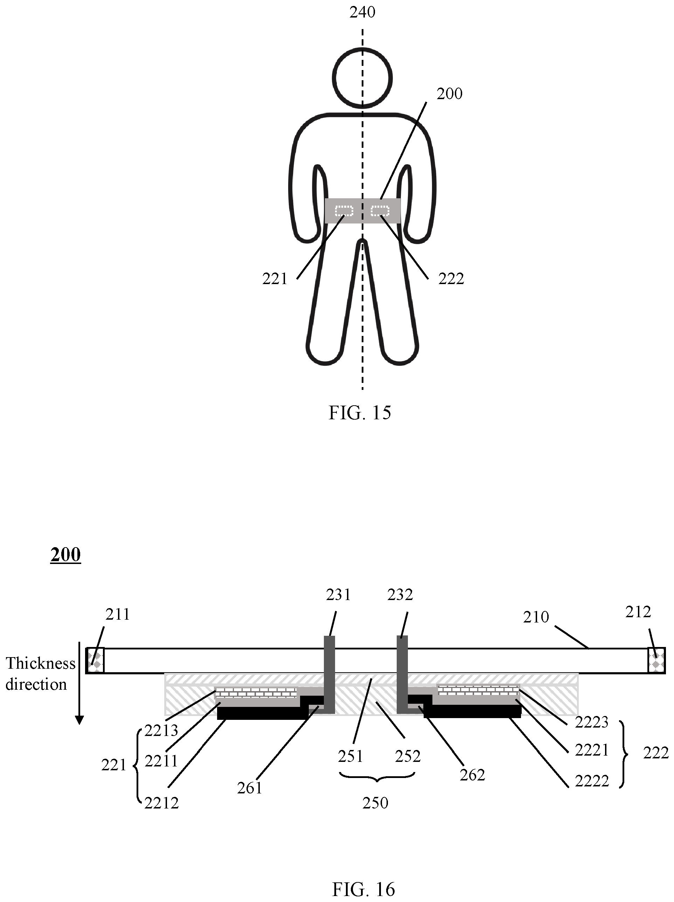

FIG. 15 is a schematic diagram illustrating positions where one or more electrodes are located on a human body according to some embodiments of the present disclosure.

In some embodiments, a first electrode and a second electrode are configured to measure electrocardiographic signals, and the first electrode and the second electrode are located on two sides of a median sagittal plane of the human body. For example, taking the one or more electrodes 220 shown in FIG. 2A as an example, the first electrode 221 and the second electrode 222 may be located on two sides of the median sagittal plane, respectively (e.g., as shown in FIG. 15, the first electrode 221 is located on a left side of a median sagittal plane 240, and the second electrode 222 is located on a right side of the median sagittal plane 240). The median sagittal plane refers to a sagittal plane located at a median position of the human body. The median sagittal plane passes through a midline of the navel and vertically divides the body into two symmetrical left and right portions.

In some embodiments, positions of the human body to which the two electrodes are positioned may be symmetric with respect to the median sagittal plane, i.e., distances between each of the two electrodes and the median sagittal plane are equal. Exemplarily, taking the two electrodes shown in FIG. 2A as an example, the first electrode 221 is located at a position on a body surface directly opposite the left ilium, and the second electrode 222 is located at a position on the body surface directly opposite the right ilium.

In some embodiments of the present disclosure, by configuring the two electrodes on the two sides of the median sagittal plane, the quality of the collected electrocardiographic signals can be effectively improved, which is beneficial to improving the signal-to-noise ratio of the electrocardiographic signals. By further configuring the two electrodes at symmetric positions on the two sides of the median sagittal plane, the quality of the collected electrocardiographic signals can be further improved.

In some embodiments, the first electrode 221 and the second electrode 222 are configured to collect electromyographic signals of a same muscle, and the first electrode 221 and the second electrode 222 are arranged at intervals along a direction of muscle fibers of the muscle. The first electrode 221 and the second electrode 222 may collect electrical potentials on a skin surface at respective positions, and a potential difference between the collected electrical potentials may be used to reflect the electromyographic signals of the muscle. In some embodiments, the first electrode 221 and the second electrode 222 may be configured to collect electromyographic signals of a target muscle of a user. The target muscle may be a single muscle or a same muscle group (e.g., a quadriceps muscle group including a rectus femoris, a vastus lateralis, a vastus medialis, and a vastus intermedius). For example, the first electrode 221 and the second electrode 222 may be arranged at intervals along a direction of muscle fibers of the target muscle and extend along an extension direction perpendicular to the direction of the muscle fibers, respectively, so as to collect electrical potentials on the skin surface at the respective positions, and a potential difference between the collected electrical potentials may be used to reflect the electromyographic signals of the target muscle. In some embodiments, when the target muscle is the single muscle, since a plurality of other muscles exist around the target muscle, the electromyographic signals collected by the one or more electrodes 220 may include electromyographic signals of the target muscle and the other muscles around the target muscle. Since the target muscle and the other muscles around the target muscle are in a same muscle group, and electromyographic signals generated by muscles in the same muscle group are relatively close, the electromyographic signals actually collected by the one or more electrodes 220 are close to the electromyographic signals of the target muscle, and an error is small. That is, the electromyographic signals actually collected by the one or more electrodes 220 may characterize the electromyographic signals of the target muscle. It should be noted that, in other embodiments, a proportion of noise (i.e., the electromyographic signals of other muscles around the target muscle) in the electromyographic signals collected by the one or more electrodes 220 may also be reduced by means of a shape design, an algorithm processing, etc., of the one or more electrodes 220, so as to improve the accuracy of the device for monitoring physiological signals. For example, the first electrode 221 and the second electrode 222 may be arranged at intervals along a direction of muscle fibers of a gastrocnemius and extend along an extension direction perpendicular to the direction of the muscle fibers, respectively. That is, the first electrode 221 and the second electrode 222 may be disposed at the gastrocnemius, arranged at intervals along a height direction of the user, and extend along an extension direction perpendicular to the height direction of the user, respectively. The first electrode 221 and the second electrode 222 collect electrical potentials on the skin surface at the respective positions, and a potential difference between the collected electrical potentials may be used to reflect the electromyographic signals of the gastrocnemius.

In some embodiments, two metal snaps are fixedly disposed on the base body, one of the two metal snaps is electrically connected to the first electrode, and the other of the two metal snaps is electrically connected to the second electrode.

In some embodiments, the two metal snaps may be connected to an external processing circuit, and the two metal snaps implement data transmission between the first electrode and the second electrode and the processing circuit (e.g., transmitting the collected physiological signals to the processing circuit). In some embodiments, the processing circuit may be detachably connected to the two metal snaps. For example, the processing circuit may be detachably connected to the two metal snaps in a magnetic attraction manner.

Exemplarily, as shown in FIG. 2B to FIG. 14, two metal snaps (a metal snap 231 and a metal snap 232) are fixedly disposed on the base body 210. The metal snap 231 is electrically connected to the first electrode 221, and the metal snap 232 is electrically connected to the second electrode 222. In some embodiments, the metal snap may penetrate through the base body 210 (and at least a portion of the waterproof layer) along a thickness direction of the base body 210 to be electrically connected to each of the one or more electrodes. For example, as shown in FIG. 14, the metal snap 231 penetrates through the base body 210, the first waterproof layer 251, and a portion of the second waterproof layer 252 along the thickness direction of the base body 210 to be electrically connected to the first electrode 221. The metal snap 232 penetrates through the base body 210, the first waterproof layer 251, and the portion of the second waterproof layer 252 along the thickness direction of the base body 210 to be electrically connected to the second electrode 222. At this time, the metal snap 231 and the metal snap 232 do not protrude from an inner surface of the second waterproof layer 252. In this setting, ends of the metal snaps are wrapped in the second waterproof layer 252, which can avoid a contact between the metal snaps and the human skin, thereby avoiding a scratch of the human skin by the metal snaps and also improving the wearing comfort. In other alternative embodiments, the metal snap 231 may also completely penetrate through the base body 210, the first waterproof layer 251, and the second waterproof layer 252 along the thickness direction of the base body 210 to be electrically connected to the first electrode 221. The metal snap 232 completely penetrates through the base body 210, the first waterproof layer 251, and the second waterproof layer 252 along the thickness direction of the base body 210 to be electrically connected to the second electrode 222. At this time, the metal snap 231 and the metal snap 232 are flush with the inner surface of the second waterproof layer 252 or protrude from the inner surface of the second waterproof layer 252. In some embodiments, each of the two metal snaps may be set as a bent structure (e.g., an L-shaped structure). After penetrating through the base body 210 (and the waterproof layer), each of the two metal snaps is bent toward a direction of a corresponding electrode to fit a surface of the corresponding electrode facing the human skin, thereby realizing an electrical connection between the two metal snaps and the electrodes. A bent portion of each of the two metal snaps (i.e., a portion of each of the two metal snaps that fits the surface of the corresponding electrode facing the human skin) may be referred to as a snap foot. In some embodiments, portions of the metal snap 231 and the metal snap 232 located on an outer surface of the base body 210 may be connected to the external processing circuit (not shown in the figure). The processing circuit may obtain electrical signals (e.g., the electromyographic signals and the electrocardiographic signals) collected by the first electrode 221 and the second electrode 222 through the metal snap 231 and the metal snap 232, and determine physiological signals (e.g., the electrocardiographic signals) and/or physiological data (e.g., an electrocardiogram) of a wearer (e.g., the monitoring object 140) based on the electrical signals.

FIG. 16 is a schematic diagram illustrating another front view of a device for monitoring physiological signals according to some other embodiments of the present disclosure. In some embodiments, a wearable body may include one or more conductive layers, the one or more conductive layers are located between two metal snaps and one or more electrodes, and the two metal snaps are electrically connected to the one or more electrodes through the one or more conductive layers. Exemplarily, as shown in FIG. 16, the wearable body 200 may include one or more conductive layers (a conductive layer 261 and a conductive layer 262). The conductive layer 261 is located between the metal snap 231 and the first electrode 221, and the metal snap 231 is electrically connected to the first electrode 221 through the conductive layer 261. The conductive layer 262 is located between the metal snap 232 and the second electrode 222, and the metal snap 232 is electrically connected to the second electrode 222 through the conductive layer 262. In some embodiments, along the thickness direction of the base body 210, the conductive layer 261 may be located between the snap foot of the metal snap 231 and the coating 2212 of the first electrode 221. The conductive layer 262 is located between the snap foot of the metal snap 232 and the coating 2222 of the second electrode 222. In some embodiments, the one or more conductive layers may include conductive yarns (e.g., silver yarns), and the one or more conductive layers are formed by weaving the conductive yarns. The one or more conductive layers are adhesively connected to the one or more electrodes and/or the two metal snaps.

By setting the conductive layers between the metal snaps and the electrodes, the stability of an electrical connection between the metal snaps and the electrodes can be improved.

The basic concepts have been described above. Obviously, to those skilled in the art, the above detailed disclosure is only an example and does not constitute a limitation to the present disclosure. Although not explicitly stated here, those skilled in the art may make various modifications, improvements, and amendments to the present disclosure. These modifications, improvements, and amendments are intended to be suggested by the present disclosure, and are within the spirit and scope of the exemplary embodiments of the present disclosure.

## Claims

1. A device for monitoring physiological signals, comprising:
a wearable body including:
one or more electrodes configured to contact human skin to collect human physiological signals, wherein each of the one or more electrodes is formed by printing conductive silicone ink on a substrate; and
a base body configured to carry the one or more electrodes and position the one or more electrodes at a target portion of a human body.

2. The device of claim 1, wherein the substrate of each of the one or more electrodes is stacked on a surface of the base body close to the human skin.

3. The device of claim 2, wherein the substrate of each of the one or more electrodes and the base body are connected by adhesion.

4. The device of claim 1, wherein the substrate of each of the one or more electrodes and the base body are connected in a side-by-side manner.

5. The device of claim 2 or 4, wherein the base body and the substrate are formed by integrated weaving of one or more yarns.

6. The device of claim 1, wherein the wearable body further includes a waterproof layer, and at least a portion of the one or more electrodes is connected to the base body through the waterproof layer.

7. The device of claim 6, wherein the waterproof layer includes a first waterproof film, the first waterproof film is stacked on a surface of the base body close to the human skin, and two side surfaces of the first waterproof film are adhesively connected to the base body and the one or more electrodes, respectively.

8. The device of claim 7, wherein the waterproof layer further includes a second waterproof film, the second waterproof film is stacked on a side of the first waterproof film close to the human skin, and the second waterproof film is adhesively connected to a peripheral side of at least a portion of the one or more electrodes.

9. The device of claim 6, wherein the waterproof layer includes a waterproof insulating yarn, the base body includes an elastic yarn, and the waterproof layer and the base body are formed by integrated weaving.

10. The device of claim 9, wherein the waterproof layer is stacked on a surface of the base body close to the human skin, and the substrate of each of the one or more electrodes is located on a side of the waterproof layer away from the base body.

11. The device of claim 9, wherein the waterproof layer and the base body are woven in a side-by-side manner, the base body is connected to a peripheral side of the waterproof layer, and the substrate of each of the one or more electrodes is located on a surface of the waterproof layer close to the human skin.

12. The device of claim 10 or 11, wherein the substrate of each of the one or more electrodes includes an insulating yarn, and the substrate and the waterproof layer are formed by integrated weaving.

13. The device of claim 9, wherein the substrate, the waterproof layer, and the base body are woven in a side-by-side manner, and the waterproof layer isolates the substrate from the base body.

14. The device of claim 6, wherein each of the one or more electrodes further includes an underlayer, and at least a portion of the underlayer is located between the waterproof layer and a portion of the substrate.

15. The device of claim 1, wherein the one or more electrodes include a first electrode and a second electrode, the first electrode and the second electrode are configured to measure electrocardiographic signals, and the first electrode and the second electrode are located on two sides of a median sagittal plane of the human body.

16. The device of claim 1, wherein the one or more electrodes include a first electrode and a second electrode, the first electrode and the second electrode are configured to collect electromyographic signals of a same muscle, and the first electrode and the second electrode are arranged at intervals along a direction of muscle fibers of the muscle.

17. The device of claim 15 or 16, wherein two metal snaps are fixedly disposed on the base body, one of the two metal snaps is electrically connected to the first electrode, the other of the two metal snaps is electrically connected to the second electrode, the two metal snaps implement data transmission between the first electrode and the second electrode and a processing circuit, and the processing circuit is detachably connected to the two metal snaps in a magnetic attraction manner.

18. The device of claim 17, wherein the wearable body further includes one or more conductive layers, the one or more conductive layers are located between the two metal snaps and the one or more electrodes, and the two metal snaps are electrically connected to the one or more electrodes through the one or more conductive layers.

19. The device of claim 1, wherein the base body is brushed.

20. The device of claim 1, wherein a conductivity of the one or more electrodes is in a range of 0.1 S/cm to 0.3 S/cm.
